(19) Europäisches Patentamt
European Patent Office
Office européen des brevets

(11) **EP 4 722 378 A1**

(12) **EUROPEAN PATENT APPLICATION**
published in accordance with Art. 153(4) EPC

(43) Date of publication:
**08.04.2026 Bulletin 2026/15**

(21) Application number: **24815599.6**

(22) Date of filing: **31.05.2024**

(51) International Patent Classification (IPC):
**C12P 21/08** *(2006.01)*

(52) Cooperative Patent Classification (CPC):
**C07K 16/00**

(86) International application number:
**PCT/JP2024/019989**

(87) International publication number:
**WO 2024/248119 (05.12.2024 Gazette 2024/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.05.2023 JP 2023090289**

(71) Applicant: **FUJIFILM Corporation Tokyo 106-8620 (JP)**

(72) Inventors:
• **NAKAI, Shinichi**
  **Kanagawa 258-8577 (JP)**

• **INADA, Atsushi**
  **Kanagawa 258-8577 (JP)**
• **SHIMONO, Katsuhiro**
  **Kanagawa 258-8577 (JP)**
• **OHIRA, Shino**
  **Kanagawa 258-8577 (JP)**
• **OHASHI, Noriyuki**
  **Kanagawa 258-8577 (JP)**
• **LI, Yuanzhong**
  **Kanagawa 258-8577 (JP)**
• **HASHIMOTO, Ichihiko**
  **Kanagawa 258-8577 (JP)**

(74) Representative: **Hoffmann Eitle Patent- und Rechtsanwälte PartmbB Arabellastraße 30 81925 München (DE)**

(54) **METHOD FOR PRODUCING PRODUCT**

(57)     An object of the present invention is to provide a method that makes it possible to prevent clogging of a membrane during culture and prevent a decrease in productivity in a production method for a product by cell culture.

According to the present invention, there is provided a production method for a product, including: culturing cells in a culture solution, in which, in a case where a poloxamer concentration in the culture solution is denoted by P (g/L), a product concentration in the culture solution is denoted by C (g/L), a hydrophobicity index SAP value of the product is denoted by S, and a ratio of a valine residue of an L chain variable region of the product is denoted by V (%), at least one of $1.5P + 3C + 0.93S \leq 378$ (Expression 1) or $P + 3.5C + 1.9V \leq 29$ (Expression 5) is satisfied.

EP 4 722 378 A1

## Description

## Technical Field

The present invention relates to a production method for a product, including culturing cells in a culture solution.

## Background Art

Cell culture is carried out for the intended purpose, for example, increasing the number of cells having useful properties or causing cells to produce a product. In producing a biopharmaceutical product such as antibodies, a perfusion culture method, in which a culture solution is continuously filtered and discharged while a fresh medium containing nutrients is continuously fed to a culture tank, is often used to improve productivity of antibodies.

For example, Patent Document 1 discloses a production method for a double-specific antibody product, the method including (i) a step of supplying at least one kind of mammalian cell capable of expressing a double-specific antibody product in a perfusion bioreactor, (ii) a step of proliferating a mammalian cell culture at a first perfusion amount until a set value of a viable cell density is reached, and (iii) a step of maintaining perfusion culture at a second perfusion amount where the step is such that the concentration of the double-specific antibody product in the bioreactor is maintained to be lower than a threshold value.

## Prior Art Documents

## Patent Documents

Patent Document 1: JP2021-505182A

## Summary of Invention

## Object to be solved by the invention

Perfusion culture has attracted attention as a method for improving productivity of antibody drugs. In the perfusion culture, a membrane filtration method is often used as a unit for continuously separating the cells, but there is a problem in that a membrane is clogged during the culture, the culture cannot be performed for a desired period, and the productivity is reduced. An object to be achieved by the present invention is to provide a method that makes it possible to prevent clogging of a membrane during culture and prevent a decrease in productivity in a production method for a product by cell culture.

## Means for solving the object

As a result of diligent studies to achieve the above objects, the inventors of the present invention found that the clogging of the membrane can be suppressed by appropriately controlling a hydrophobicity index SAP of the antibody, an antibody concentration in the culture solution, and a poloxamer concentration in the culture solution, whereby the present invention was completed.

That is, according to the present invention, the following inventions are provided.

<1> A production method for a product, comprising:

culturing cells in a culture solution,
in which, in a case where a poloxamer concentration in the culture solution is denoted by P (g/L),
a product concentration in the culture solution is denoted by C (g/L),
a hydrophobicity index SAP value of the product is denoted by S, and
a ratio of a valine residue of an L chain variable region of the product is denoted by V (%),
at least one of

$$1.5P + 3C + 0.93S \leq 378 \quad \text{(Expression 1)}$$

or

$$P + 3.5C + 1.9V \leq 29 \text{ (Expression 5)}$$

is satisfied.

<2> The production method for a product according to <1>,
in which the poloxamer concentration P (g/L) satisfies

$$1.5 \leq P \leq 15 \text{ (Expression 2)}.$$

<3> The production method for a product according to <1> or <2>,
in which the product concentration C (g/L) satisfies

$$0.2 \leq C \leq 10 \text{ (Expression 3)}.$$

<4> The production method for a product according to any one of <1> to <3>,
in which S, which is the hydrophobicity index SAP value, satisfies

$$300 \leq S \leq 394 \text{ (Expression 4)}.$$

<5> The production method for a product according to any one of <1> to <4>,
in which the ratio V (%) satisfies

$$0 \leq V \leq 9 \text{ (Expression 6)}.$$

<6> The production method for a product according to any one of <1> to <5>, in which the culturing is perfusion culture.
<7> The production method for a product according to <6>, in which the perfusion culture includes a cell separation step of separating the cells by a filtration membrane.
<8> The production method for a product according to <7>, in which the cell separation step is performed by an ATF method which is an alternating tangential flow.
<9> The production method for a product according to any one of <6> to <8>, in which, in the perfusion culture, the product is produced in a certain period while maintaining a target viable cell density substantially constant after reaching the viable cell density in the culturing.
<10> The production method for a product according to <9>, in which the viable cell density maintained constant is 30 $\times$ 10$^6$ cells/ml or more and 400 $\times$ 10$^6$ cells/ml or less.
<11> The production method for a product according to any one of <6> to <10>, in which a perfusion ratio in the perfusion culture is 0.3 or more and 3.0 or less.
<12> The production method for a product according to any one of <1> to <11>, in which a period of the culturing is 14 days or more and 200 days or less.
<13> The production method for a product according to any one of <1> to <12>, in which the cell is a CHO cell.
<14> The production method for a product according to any one of <1> to <13>, in which the product is a protein.
<15> The production method for a product according to <14>, in which the protein is an antibody.
<16> The production method for a product according to <15>, in which a light chain structure of the antibody is a κ type.

**Effect of the invention**

According to the production method for a product according to the present invention, it is possible to prevent the clogging of the membrane during culture and prevent the decrease in the productivity.

**BRIEF DESCRIPTION OF THE DRAWINGS**

[FIG. 1]FIG. 1 shows a cell culture device.
[FIG. 2] FIG. 2 shows measurement results of a viable cell density.
[FIG. 3] FIG. 3 shows measurement results of a reciprocating pressure of ATF.
[FIG. 4] FIG. 4 shows measurement results of an antibody permeation rate.

[FIG. 5] FIG. 5 shows measurement results of titer.

**Embodiments for carrying out the invention**

Hereinafter, the contents of the present invention will be described in detail. In the present specification, a numerical value range indicated using "to" means a range including numerical values described before and after "to" as a minimum value and a maximum value, respectively.

A production method for a product according to an embodiment of the present invention is a production method for a product, including:

culturing cells in a culture solution,
in which, in a case where a poloxamer concentration in the culture solution is denoted by P (g/L),
a product concentration in the culture solution is denoted by C (g/L),
a hydrophobicity index SAP value of the product is denoted by S, and
a ratio of a valine residue of an L chain variable region of the product is denoted by V (%),
at least one of

$$1.5P + 3C + 0.93S \leq 378 \quad \text{(Expression 1)}$$

or

$$P + 3.5C + 1.9V \leq 29 \text{ (Expression 5)}$$

is satisfied.

The production method for a product according to the embodiment of the present invention can be suitably used as a production method for a product by cell culture, which is used for a biopharmaceutical product, regenerative medicine, or the like.

Preferably, P, C, and S satisfy

$$340 \leq 1.5P + 3C + 0.93S \leq 370 \quad \text{(Expression 1A)}.$$

Preferably, P, C, and V satisfy

$$P + 3.5C + 1.9V \leq 23 \text{ (Expression 5A)}.$$

By setting $1.5P + 3C + 0.93S$ to 378 or less or setting $P + 3.5C + 1.9V$ to 29 or less, the aggregation of the antibody and the poloxamer can be suppressed, and the membrane clogging can be suppressed.

The poloxamer concentration P (g/L) in the culture solution preferably satisfies

$$1.5 \leq P \leq 15 \text{ (Expression 2)}.$$

In a case where the poloxamer concentration P is set to 1.5 g/L or more, the cells suitably proliferate due to the cell protective effect of the poloxamer, and the occurrence of cell debris can also be suppressed. By setting the poloxamer concentration P to 15 g/L or less, the aggregation of the antibody and the poloxamer can be suppressed, and the membrane clogging can be suppressed.

From the viewpoint of suppressing the aggregates, the poloxamer concentration is preferably 1.5 g/L or more and less than 6 g/L (more preferably 2.0 g/L or more and 5.8 g/L or less, still more preferably 3.0 g/L or more and 5.5 g/L or less, and even still more preferably 4.0 g/L or more and 5.5 g/L or less).

From the viewpoint of the cell protective effect, the poloxamer concentration is preferably 6 g/L or more and 15 g/L or less (more preferably 7 g/L or more and 14 g/L or less, still more preferably 8 g/L or more and 13 g/L or less, and even still more preferably 9 g/L or more and 12 g/L or less). In this case, the amount of the aggregates increases, but it is effectively suppressed by the present invention.

The culture solution contains both cells and a culture supernatant.

The poloxamer concentration in the culture solution can be measured by the following method. A culture solution sample is extracted from the culture tank, and the cells are filtered to collect a filtrate. 1.17 g of maleic acid (guaranteed reagent, manufactured by FUJIFILM Wako Pure Chemical Corporation) is added to 50 ml of deuterium oxide and dissolved. 0.4 ml of heavy water in which 20, 50, 100, or 200 mg of Kolliphor P188 (manufactured by BASF SE) has been dissolved is added to 1.6 ml of heavy water containing maleic acid, and the mixture is measured by 1H NMR (400 MHz, Ascend 400, manufactured by Bruker Corporation) with 64 accumulations. A calibration curve is drawn at peak 1 (near 0.8 to 1.1 ppm) and peak 2 (near 3.4 to 3.5 ppm) of the poloxamer with reference to 6.2 ppm, which is the peak of the maleic acid. 0.4 ml of the culture supernatant is added to 1.6 ml of heavy water containing maleic acid, and the mixture is measured by 1H NMR with 64 accumulations to perform quantification from the area ratio of peak 1 or peak 2 to the maleic acid. Either peak 1 or peak 2 is used depending on the level of impurities.

The product concentration C (g/L) preferably satisfies

$$0.2 \leq C \leq 10 \ (\text{Expression 3}).$$

In a case where the product concentration C is set to 0.2 g/L or more, the recovery amount of the antibody is increased, and high productivity can be maintained. By setting the product concentration C to 10.0 g/L or less, the aggregation of the antibody and the poloxamer can be suppressed, and the membrane clogging can be suppressed.

The product concentration C is more preferably 0.2 g/L or more and 10.0 g/L or less, still more preferably 0.5 g/L or more and 5.0 g/L or less, and particularly preferably 1.0 g/L or more and 3.5 g/L or less.

The product can be purified by a purification treatment. The obtained product can be purified to high purity. For the separation and purification of the product, the separation and purification methods that are used for general proteins may be used. For example, it is possible to carry out separation and purification of a product by appropriately selecting and combining means such as a chromatography such as affinity chromatography, a filter, ultrafiltration, salting out, dialysis, sodium dodecyl sulfate (SDS) polyacrylamide gel electrophoresis, isoelectric focusing electrophoresis, and the like, which are not limited thereto. The concentration of the product obtained as above can be measured according to absorbance measurement, enzyme-linked immunosorbent assay (ELISA), or the like.

In addition, in a case where the product is an antibody, the product concentration can be measured by extracting the sample from the culture tank, filtering the cells with Whatman (pore size: 0.2 $\mu$m, diameter: 25 mm) manufactured by Cytiva, and then measuring the filtrate with Cedex Bio manufactured by F. Hoffmann-La Roche, Ltd.

S, which is the hydrophobicity index SAP value, preferably satisfies

$$300 \leq S \leq 394 \ (\text{Expression 4}).$$

In a case where the SAP value is 300 or more, a protein structure that can be suitably used for therapy can be obtained. By setting the SAP value to 394 or less, the aggregation of the antibody and the poloxamer can be suppressed, and the membrane clogging can be suppressed.

The SAP value is more preferably 350 or greater and less than 394 and still more preferably 350 or greater and 390 or less.

The spatial aggregation propensity (SAP) value is an index indicating how much hydrophobic amino acids (for example, valine, leucine, and alanine) are aggregated on the surface of the protein.

The SAP value can be obtained by the following procedure.

(1) The three-dimensional structure of the protein is predicted. As software for predicting the three-dimensional structure of the protein, AlphaFold2 can be used (in Examples of the present application, training data up to July 14, 2021 was used). In addition, software such as MOE, Schrodinger, and BIOVIA Discovery Studio can also be used in addition to AlphaFold2.

In the case of the antibody, the Fab and Fc regions can also be predicted separately, or the Fab region and the Fc region can be predicted at once. In Examples of the present application, the Fab and Fc regions were predicted separately.

(2) The Fab and the Fc are aligned with reference to the crystal structure of 1hzh in the Protein Data Bank for the IgG1 antibody and to a structure created by modifying the crystal structure of 1hzh for the IgG2 and IgG4 antibodies. For the alignment calculation of the structure, software such as MOE, Schrodinger, BIOVIA Discovery Studio, Pymol, and TM-align can also be used.

(3) Structural optimization using molecular dynamics is performed while applying constraints between the S-S of CYS according to the structure of the antibody, to form the bonds.

(4) The molecular dynamics calculation is performed to acquire the stable structure of the antibody in equilibrium. As software for the bond formation of the CYS and the molecular dynamics calculation, Amber, Ambertools, Gromacs, and the like can be used.

(5) The SAP value is calculated according to the following paper.

Naresh Chennamsetty (2009), Design of therapeutic proteins with enhanced stability, Proc Natl Acad Sci USA. 2009 Jul 21; 106 (29): 11937-42

As the amino acid-specific hydrophobicity value residue hydrophobicity, the numbers described in the following paper were used.

Shaun D. Black (1991), Development of hydrophobicity parameters to analyze proteins which bear post- or cotranslational modifications, Analytical Biochemistry Volume 193, Issue 1, 15 February 1991, Pages 72 to 82

As software for the calculation of the SAP value, MOE, BIOVIA Discovery Studio, and the like can be used.

The SAP value of each antibody described in Table 1 of Examples was calculated by the above-described method.

The aggregation risk can be predicted by the total of the SAP.

The ratio V (%) of a valine residue of an L chain variable region of the product preferably satisfies

$$0 \le V \le 9 \text{ (Expression 6)}.$$

By setting V to 9 or less, the aggregation of the antibody and the poloxamer can be suppressed, and the membrane clogging can be suppressed.

V is more preferably 0 or more and 9 or less, still more preferably 0 or more and 8 or less, and most preferably 0 or more and 7 or less.

V is an indicator of the ratio of the valine residue, which is a hydrophobic amino acid, in the L chain variable region.

V can be obtained by the following procedure.

The L chain variable region of the antibody is defined as a region obtained by excluding 70 residues from the C-terminus (side having a carboxyl group at the end) of the L chain of the antibody, and the ratio (%) of the number of valine residues to the number of amino acid residues in the region is defined as the value of V. In a case where the left and right chains are different as in a bispecific antibody, the calculation is performed for each of the left and right chains. In this case, aggregation does not occur as long as both of the left and right chains satisfy the condition. Aggregation occurs in a case where one of the left chain or the right chain does not satisfy the condition.

In addition, in a case where the L chain is originally configured by linking two or more different L chains of antibodies, that is, for example, in a special case where the original L chain variable region is introduced at two or more sites in the L chain, a region obtained by excluding 70 residues from the C-terminus of the L chain in the entire length of the L chain is defined as the L chain variable region, in the same manner as the calculation method for the normal antibody described above, instead of calculating the valine residue for the original L chain variable region, and the ratio (%) of the number of valine residues to the number of amino acid residues in the region is defined as the value of V.

V of each antibody described in Table 1 of Examples was calculated by the above-described method.

The aggregation risk can be predicted by the valine ratio V of the L chain variable region.

The culture in the present invention is preferably perfusion culture.

The perfusion culture is a culture method in which a fresh culture medium is added and at the same time the used culture medium is removed. In general, the perfusion culture makes it possible to achieve a high viable cell density. A typical perfusion culture begins with a batch culture start-up lasting 1 or 2 days, thereafter a fresh supply culture medium is added to the culture continuously, stepwise, and/or intermittently, and the used culture medium is removed at the same time. In the perfusion culture, methods such as sedimentation, centrifugation, and filtration can be used to remove the used culture medium while maintaining the viable cell density. The advantage of the perfusion culture is that the culture in which a target protein is produced is maintained for a long period as compared with the batch culture method or the fed-batch culture.

Perfusion may be any form of being continuous, stepwise, intermittent, or a combination thereof. A continuous form is preferable. Animal cells are retained in the culture and the used culture medium that is removed may substantially not include cells or may have much fewer cells than the culture. A product that is expressed by cell culture can be retained in the culture or recovered by the selection of the membrane pore diameter.

The period of the perfusion culture is preferably 5 days or more and 300 days or less, more preferably 14 days or more and 200 days or less, still more preferably 14 days or more and 100 days or less, and still more preferably 20 days or more and 90 days or less. It is preferable for the number of days of the culture to be 5 days or more since the obtained cell number is large and the production amount of a product is large in a case where the cells produce the product. It is preferable for the number of days of the culture to be 300 days or less from the viewpoint of preventing clogging of the filtration membrane used in the perfusion culture and preventing contamination.

It is preferable that the perfusion culture includes a cell separation step of separating the cells by a filtration membrane. The cell separation step is preferably performed by an ATF method which is an alternating tangential flow.

The perfusion ratio is not particularly limited; however, it is preferably 0.3 vvd or more and 3.0 vvd or less, more preferably 0.6 vvd or more and 2.5 vvd or less, and still more preferably 0.8 vvd or more and 2.0 vvd or less.

By setting the perfusion ratio to 0.3 or more, the antibody can be prevented from being at a high concentration, the aggregation of the antibody and the poloxamer can be suppressed, and the membrane clogging can be suppressed. By setting the perfusion ratio to 3.0 or less, the amount of the culture medium used can be suitably suppressed.

The vvd regarding the perfusion ratio means "volume of drained culture solution/volume of culture solution in culture container/day". It is noted that the perfusion ratio described herein is a parameter different from "(iv) driving a pump for draining the culture solution at a rate of 0.3 vvd to 3 vvd in a case where the electrostatic capacity of the culture solution exceeds the target" in one example of the bleeding.

The extraction of the culture solution from the culture container can usually be carried out using a pump, but other available liquid feeding units may be used. The culture solution extracted from the culture container is subjected to treatments such as product recovery and removal of the dead cells. The culture solution extracted from the culture container may be partially discarded or returned to the culture container after treatments such as product recovery and removal of the dead cells. In a case where a loss of the culture medium occurs due to the above treatments, the loss can be compensated, for example, by supplying a fresh culture medium to the culture container.

The perfusion culture preferably includes producing the product in a certain period while maintaining a target viable cell density substantially constant after reaching the viable cell density in the culturing.

The "maintaining the viable cell density substantially constant" means that the viable cell density is maintained within ±20% of the target viable cell density, preferably within ±10%, more preferably within ±5%, and still more preferably within ±3%.

The viable cell density maintained substantially constant is preferably $30 \times 10^6$ cells/ml or more and $400 \times 10^6$ cells/ml or less, more preferably $30 \times 10^6$ cells/ml or more and $300 \times 10^6$ cells/ml or less, still more preferably $80 \times 10^6$ cells/ml or more and $240 \times 10^6$ cells/ml or less, still more preferably $80 \times 10^6$ cells/ml or more and $200 \times 10^6$ cells/ml or less, even still more preferably $80 \times 10^6$ cells/ml or more and $160 \times 10^6$ cells/ml or less, even still more preferably $100 \times 10^6$ cells/ml or more and $160 \times 10^6$ cells/ml or less, and particularly preferably $100 \times 10^6$ cells/ml or more and $120 \times 10^6$ cells/ml or less. M may be used to denote $10^6$.

By setting the viable cell density to $30 \times 10^6$ cells/ml or more, the recovery amount of the antibody is increased, and high productivity can be maintained. By setting the viable cell density to $400 \times 10^6$ cells/ml or less, the aggregation of the antibody and the poloxamer can be suppressed, and the membrane clogging can be suppressed.

The viable cell density can be measured using a commercially available measuring device such as a Cell Viability Analyzer Vi-cell XR manufactured by Beckman Coulter, Inc., and as an example, it can be measured by using Vi-cell XR 2.04 as the Vi-cell software and setting the Min diameter to 6 μm and the Max diameter to 50 μm as parameters at the time of measurement. In addition, the measurement parameters can be set as follows.

    Cell brightness: 85%
    Cell sharpness: 100
    Viable cell spot brightness: 75%
    Viable cell spot area: 5%
    Minimum circularity: 0
    Decluster degree: Medium

In the present invention, it is preferable to perform cell bleeding (also referred to as bleeding) of discharging a culture solution containing cells to an outside of a system and adding a culture medium to maintain the viable cell density substantially constant.

The cell bleeding ratio in the cell bleeding is preferably 0.10 to 0.80 vvd, and more preferably 0.15 to 0.60 vvd.

By setting the cell bleeding ratio to 0.10 vvd or more, the proliferation can be appropriately maintained, and the antibody quality is stably maintained. By setting the cell bleeding ratio to 0.80 vvd or less, the culture medium use amount can be suppressed, and the cost can be reduced.

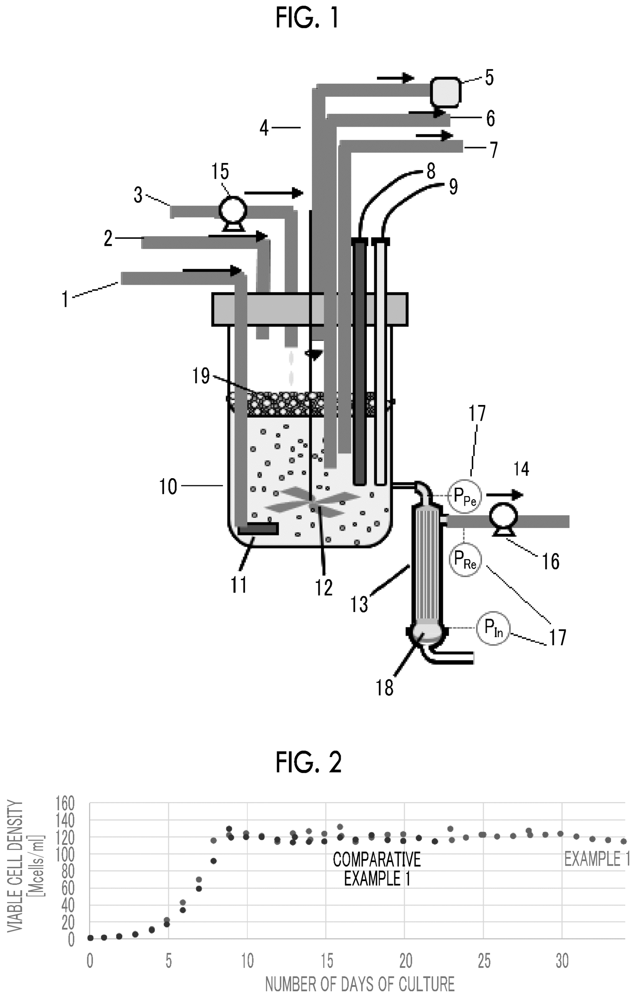

The bleeding is sufficient to be such that the culture solution containing cells can be extracted from the culture container, and the method thereof is not particularly limited. However, for example, as shown in FIG. 1, a pipe for discharging the culture solution is inserted into the culture solution, and the culture solution can be discharged from the culture container through the pipe.

Preferably, in the bleeding, the culture solution may be bled while an electrostatic capacity of the culture solution is subjected to in-line measurement. The in-line measurement is to measure any measurement value indicating a state of the culture solution while carrying out cell culture. In order to subject the electrostatic capacity to in-line measurement, for example, as shown in FIG. 1, it is sufficient to insert an electrostatic capacity sensor into the culture solution and subject the electrostatic capacity of the culture solution to in-line measurement while carrying out culture.

Preferably, the bleeding may be automatically controlled. The automatic control means that the start or stop of the bleeding is automatically controlled based on any measurement value indicating any state of the culture solution. For example, the start and stop of the bleeding can be automatically controlled based on the measurement value of the electrostatic capacity of the culture solution.

Preferably, the bleeding may be carried out such that a variation of the electrostatic capacity of the culture solution, where the electrostatic capacity is subjected to in-line measurement, is ±20% or less with respect to an average value of an electrostatic capacity during the production period. The variation of the electrostatic capacity of the culture solution, where the electrostatic capacity is subjected to in-line measurement, is more preferably ±15% or less with respect to an average value of an electrostatic capacity during the production period, still more preferably ±12% or less with respect to the average value of the electrostatic capacity during the production period, and particularly preferably ±8% or less with respect to the average value of the electrostatic capacity during the production period. The lower limit of the variation of the electrostatic capacity of the culture solution is not particularly limited, and it is sufficient to be 0% or more; however, a variation of substantially 0.1% or more occurs in consideration of the variation of daily cell proliferation.

One exemplary bleeding can be carried out by a step including:

(i) setting an electrostatic capacity serving as a target;
(ii) setting a weight control value of the culture container;
(iii) measuring the electrostatic capacity of the culture solution at a period of 0.1 seconds or less;
(iv) driving a pump for draining the culture solution at a rate of 0.3 vvd to 3 vvd in a case where the electrostatic capacity of the culture solution exceeds the target (here, vvd means "volume of drained culture solution/volume of culture solution/day");
(v) automatically supplying a culture medium to the culture container while measuring a mass of the culture solution such that a variation of a liquid amount of the culture solution is within 10%; and
(vi) stopping the pump for draining the culture solution in a case where the electrostatic capacity of the culture solution is smaller than the target by 0.01% or more.

FIG. 1 shows one example of a cell culture device that can be used in the cell culture in the present invention. In FIG. 1, a culture container 10 is a container that accommodates a culture solution containing cells. Cells are cultured in the culture solution in the inside of the culture container 10.

Oxygen and air are sent from a sparger air supply pipe 1, and the oxygen and the air are introduced into the culture solution through a sparger 11 having a hole diameter of 20 $\mu$m. The dissolved oxygen concentration inside the culture solution can be adjusted by the sparger 11. The sparger is not particularly limited; however, it is possible to use, for example, a sparger in which an average hole diameter of a gas release unit is 1 $\mu$m or more and 300 $\mu$m or less and which releases a gas containing 30% by volume or more of oxygen.

Air and carbon dioxide are introduced from an air supply pipe 2 to an upper part of the culture solution in the culture container.

The culture medium is supplied to the culture container from a culture medium supply pipe 3. A culture medium supply pump 15 is provided in the culture medium supply pipe 3.

A gas exhaust pipe 4 is a pipe for gas exhaust, and a gas exhaust filter 5 is connected to a terminal of the gas exhaust pipe 4.

A sampling tube 6 is a pipe for collecting (sampling) the culture solution.

A bleeding tube 7 is a pipe for extracting (bleeding) the culture solution.

An electrostatic capacity sensor 8 is mounted to come into contact with the culture solution in the culture container.

A dissolved oxygen sensor 9 is mounted to come into contact with the culture solution in the culture container.

A stirring member having a stirring blade 12 may be provided inside the culture container 10. In a case where the stirring blade 12 is rotated, the culture solution inside the culture container 10 is stirred, and the homogeneity of the culture solution is maintained. In a case where the culture solution is stirred by the stirring blade 12, the bubbles released by the sparger are also stirred. The position of the stirring member having a stirring blade, the size of the stirring blade, and the like are not particularly limited and may be designed depending on the cell kind to be used, the amount of the culture solution, the amount of oxygen to be supplied, or the position, number, size, or the like of the sparger. Further, in order to quickly stir bubbles coming out of the sparger and suppress coalescence of the bubbles, it is preferable to dispose the stirring blade 12 at a position close to the sparger. FIG. 1 shows a state in which foam 19 is present.

A perfusion device 13 is connected to a lower part of the culture container 10. In addition, the culture solution inside the culture container 10 passes through the perfusion device 13 (hollow fiber MF membrane), and the permeated solution containing the cell product is extracted from the culture container 10 by a delivery pump 16. The flow of the permeated solution is indicated by an arrow 14. A pressure gauge 17 and a diaphragm pump 18 may be installed in the perfusion device 13.

In the present invention, a cell suspension extracted from the culture container may be allowed to pass through a separation membrane to separate the cell suspension into a cell-containing liquid and a permeated solution. This operation can be carried out using a perfusion device. In this operation, the cell suspension extracted from the culture container is separated into a cell-containing liquid having a cell density higher than that of the cell suspension and a permeated solution having a cell density lower than that of the cell suspension. The cell density can be measured by a live/dead cell analyzer Vi-CELL XR, manufactured by Beckman Coulter, Inc.

The membrane separation treatment step described above is preferably tangential filtration, more preferably alternating tangential flow (ATF) or tangential flow, and most preferably ATF. Examples of the filter that can perform ATF include SuATF10-S02PES, F2 RF02PES, or the like, manufactured by Repligen Corporation.

As the culture medium that is used for cell culture, a culture medium that is generally used for culturing animal cells can be used. For example, CD OptiCHO (manufactured by Thermo Fisher Scientific, Inc.), Dulbecco's modified Eagle medium (DMEM), Eagle minimum essential medium (MEM), RPMI-1640 medium, RPMI-1641 medium, F-12K medium, Ham's F12 medium, Iscove's modified Dulbecco's medium (IMDM), McCoy's 5A medium, Leibovitz's L-15 medium, and EX-CELL (trade mark) 300 series (JRH Biosciences), CHO-S-SFMII (Invitrogen), CHO-SF (Sigma-Aldrich Co. LLC), CD-CHO (Invitrogen), ISCHO-V (FUJIFILM Irvine Scientific), PF-ACF-CHO (Sigma-Aldrich Co. LLC), and the like can be used. Alternatively, a homemade culture medium may be used.

Serum such as fetal calf serum (FCS) may be added to the culture medium, or serum may not be added thereto. The culture medium may be supplemented with additional components such as an amino acid, salts, a sugar, a vitamin, a hormone, a growth factor, a buffer solution, an antibiotic, a lipid, a trace element, and a hydrolysate of a plant protein. A protein-free culture medium can also be used.

Although the pH of the culture medium varies depending on the cells to be cultured, the culture medium generally has a pH of 6.0 to 8.0, preferably has a pH of 6.4 to 7.6, and more preferably has a pH of 6.7 to 7.4.

The culture temperature is generally 30°C to 40°C, preferably 32°C to 39°C, and more preferably 36°C to 38°C, and the culture temperature may be changed during the culture.

The culture can be carried out in an atmosphere having a $CO_2$ concentration of 0% to 40% by volume, preferably 2 to 25% by volume, and more preferably 3 to 20% by volume.

The amount of the culture solution is preferably 1 L or more, more preferably 50 L or more, and still more preferably 200 L or more.

The culture period is not particularly limited, but it is generally 5 days or more and 300 days or less, preferably 14 days or more and 200 days or less, more preferably 14 days or more and 100 days or less, and still more preferably 20 days or more and 90 days or less.

In the culture, the culture medium can be replaced, aerated, and stirred as necessary. In a case of carrying out stirring of the culture, the rotation speed of stirring is not particularly limited; however, the stirring power per unit volume is generally 10 to 300 kW/m$^3$, preferably 20 to 200 kW/m$^3$, and more preferably 30 to 100 kW/m$^3$.

The dissolved oxygen concentration in the culture solution can be appropriately set and is not particularly limited; however, it is 20% to 150%, preferably 30% to 120%, and more preferably 50% to 100% in a case where the saturated dissolved oxygen concentration in the liquid at 37°C in air at 1 atm is set to 100%.

The cell culture can be carried out using a cell culture device having the configuration described above in the present specification. The cell culture device may be any one of a fermenter tank type culture device, an air lift type culture device, a culture flask type culture device, a spinner flask type culture device, a microcarrier type culture device, a fluidized bed type culture device, a hollow fiber type culture device, a roller bottle type culture device, a filling tank type culture device, or the like. The culture container is preferably a single-use culture tank from the viewpoint of homogenizing the culture environment or the like. From the viewpoint of homogenization of the culture environment and the like, the cell culture device may be a single-use culture tank.

The viscosity of the culture solution is preferably 1.2 mPa·s or more and less than 15 mPa·s, more preferably 1.4 mPa·s or more and less than 12 mPa·s, and particularly preferably 1.6 mPa·s or more and less than 10 mPa·s.

In the present invention, a pH adjusting agent may be added during the culture. From the viewpoint of the clogging of the filtration filter flow channel, a pH adjusting agent to be added to the culture tank is such that an adding amount thereof per day is preferably 8 mmol/day/L or less and more preferably 7 mmol/day/L or less. Here, it is not necessary to add a pH adjusting agent.

The pH adjusting agent is not particularly limited; however, it is preferably an aqueous $Na_2CO_3$ solution, an aqueous NaOH solution, or an aqueous $NaHCO_3$ solution, and it is more preferably an aqueous $NaHCO_3$ solution. The pH adjusting agent may be added alone or may be added by being mixed with a culture medium or an anti-foaming agent. In order to avoid the local variation of pH in the culture solution due to the addition of the pH adjusting agent, it is preferable that the pH adjusting agent is added by being mixed with a culture medium.

The kind of the cell in the present invention is not particularly limited; however, examples thereof include eukaryotic cells such as an animal cell, a plant cell, and yeast, prokaryotic cells such as Bacillus subtilis, and Escherichia coli. The cell is preferably an animal cell (more preferably a mammalian cell) or an insect cell, and it is most preferably a mammalian cell. The cell may be a primary cell or a cell established as a cell line.

Examples of the cell include a Chinese hamster ovary (CHO) cell, a HEK cell (a cell derived from the human embryonic kidney), a BHK cell, a 293 cell, a C127 cell, a myeloma cell (such as an NS0 cell), a PerC6 cell, an SP2/0 cell, a hybridoma cell, a COS cell (a cell derived from the kidney of the African green monkey), a 3T3 cell, a HeLa cell, a Vero cell (a renal epithelial cell of the African green monkey), a MDCK cell (a cell derived from a canine kidney renal tubular epithelial cell), a PC12 cell, and a WI38 cell. The cell may be a stem cell such as an embryonic stem cell (ES cell) or an induced pluripotent stem cell (iPS cell). Among these, a CHO cell, a HEK cell, a BHK cell, or a hybridoma is preferable, where a CHO cell or a HEK cell is more preferable, and a CHO cell is most preferable. The CHO cell is widely used for the production of recombinant proteins such as a cytokine, a coagulation factor, and an antibody. It is preferable to use a CHO cell deficient in dihydrofolate reductase (DHFR), and as a DHFR-deficient CHO cell, it is possible to use, for example, CHO-DG44.

It is preferable that the cell viability is high; however, it is preferably 80% or more, more preferably 85% or more, particularly preferably 90% or more, and most preferably 95% or more.

These cells may be cells into which a foreign gene encoding a protein desired to be expressed (for example, an antibody) has been introduced. The cell is preferably a cell that produces an antibody. An expression vector can be used for introducing a foreign gene encoding a protein desired to be expressed, into a cell. An expression vector containing a DNA encoding a protein desired to be expressed, an expression control sequence (for example, an enhancer, a promoter, a terminator, or the like), and a selection marker gene as desired is introduced into a cell, whereby it is possible to prepare a cell into which a foreign gene encoding a protein desired to be expressed is introduced. The expression vector is not particularly limited and can be appropriately selected and used depending on the kind, use application, and the like of the cell.

As the promoter, it is possible to use any promoter of which the function can be exhibited in mammalian cells. Examples thereof include a promoter of the immediate early (IE) gene of the cytomegalovirus (CMV), an early promoter of SV40, a retrovirus promoter, a metallothionein promoter, a heat shock promoter, an SR$\alpha$ promoter, and a promoter and enhancer of the Moloney murine leukemia virus. In addition, an enhancer of the IE gene of human CMV may be used together with the promoter.

As the selection marker gene, it is possible to use, for example, a drug resistance gene (a neomycin resistance gene, a DHFR gene, a puromycin resistance gene, a blasticidin resistance gene, a hygromycin resistance gene, a cycloheximide resistance gene), or a fluorescence gene (a gene encoding a green fluorescent protein GFP or the like).

The method of introducing an expression vector into a cell is not particularly limited, and it is possible to use, for example, a calcium phosphate method, an electroporation method, a liposome method, a gene gun method, or a lipofection method.

The production method for a product according to the embodiment of the present invention includes culturing a cell using a cell culture device to produce a product from the cell.

According to the present invention, there is provided a product that is produced by the production method for a product according to the embodiment of the present invention.

In the present invention, the kind of product is not particularly limited; however, the product is preferably a protein. Examples of the product include a recombinant polypeptide chain, a recombinant secreted polypeptide chain, an antigen-binding protein, an antibody (for example, a human antibody, a humanized antibody, a chimeric antibody, a mouse antibody, or a bispecific antibody), an Fc fusion protein, a fragmented immunoglobulin, and a single-chain antibody (scFv). In addition, it may be, in addition to the above, an adenovirus, an adeno-associated virus, a lentivirus, or the like.

The product is preferably an antibody, and more preferably a human antibody, a humanized antibody, a chimeric antibody, or a mouse antibody. Examples of the fragmented immunoglobulin include Fab, F(ab')2, and Fv. The class of the antibody is also not particularly limited, and it may be any class of IgG such as IgG1, IgG2, IgG3, or IgG4, IgA, IgD, IgE, or IgM. However, IgG or IgM is preferable in a case of being used as a medicine. The light chain structure of the antibody may be any of a $\kappa$ type or a $\lambda$ type, but is preferably a $\kappa$ type.

The human antibody includes all antibodies having one or a plurality of variable and constant regions induced from human immunoglobulin sequences. In one embodiment, all variable and constant domains are induced from human immunoglobulin sequences (complete human antibodies).

In a case of being administered to a human subject, the humanized antibody has a sequence different from a sequence of an antibody induced from a non-human species by substitution, deletion, and/or addition of one or a plurality of amino acids so that there is a low possibility for the humanized antibody to induce an immune response and/or so that induction of a severe immune response is reduced as compared with the antibody of non-human species. In one example, specific amino acids in a framework and constant domains of heavy chains and/or light chains of an antibody of non-human species are mutated to produce a humanized antibody. In another example, a constant domain from a human antibody is fused to a variable domain of an antibody of a non-human species.

The chimeric antibody is an antibody in which variable regions and constant regions having origins different from each other are linked. For example, an antibody consisting of variable regions of heavy chains and light chains of a mouse antibody and consisting of constant regions of heavy chains and light chains of a human antibody is a mouse/human heterologous chimeric antibody. It is possible to prepare a recombinant vector expressing a chimeric antibody by linking a DNA encoding variable regions of a mouse antibody and a DNA encoding constant regions of a human antibody and then incorporating the linked DNA into an expression vector. It is possible to acquire a chimeric antibody produced during the culturing by culturing a recombinant cell transformed with the above vector and expressing the incorporated DNA.

The bispecific antibody is an antibody that recognizes two kinds of antigenic specificity, which are different from each other. Various forms of bispecific antibodies are present. As a method of preparing a bispecific antibody, it has been reported a method of preparing a bispecific antibody by binding two immunoglobulin molecules by using a crosslinking agent such as N-succinimidyl 3-(2-pyridyldithiol)propionate or S-acetylmercaptosuccinic acid anhydride, a method of preparing a bispecific antibody by binding Fab fragments of immunoglobulin molecules to each other, and the like. In addition, a bispecific antibody can be expressed by introducing a gene encoding the bispecific antibody into a cell.

The Fc fusion protein indicates a protein having an Fc region and includes an antibody.

The Fab is a monovalent fragment having VL, VH, CL, and CH1 domains.

The F(ab')2 is a divalent fragment having two Fab fragments bound by a disulfide crosslinking at a hinge region.

The Fv fragment has VL and VH domains of a single arm of an antibody.

The single-chain antibody (scFv) is an antibody in which VL and VH regions are joined through a linker (for example, a synthetic sequence of amino acid residues) to form a continuous protein chain, where the linker is long enough to allow the protein chain to fold for itself and form a monovalent antigen binding site.

The antibody is not particularly limited; however, examples thereof include an anti-IL-6 receptor antibody, an anti-IL-6 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, and an anti-VLA4 antibody.

Regarding the product recovery, the culture solution may be simply recovered. For example, a liquid obtained by removing at least a part of the cells from the culture solution using a filter or a centrifuge may be recovered, and a known method is used without particular limitation. In a case of desiring to improve the purity of the product, change the solvent of the product, or change the form of the product to, for example, a powder form, the culture solution or the liquid can be subjected to further treatment.

In addition, a part of the culture solution can be recovered while being perfused, or a part of the culture solution can be recovered as a liquid which is obtained by removing at least a part of cells from the part of the culture solution, which is recovered by using a filter or a centrifuge while being perfused.

As described above in the present specification, the product can be purified by a purification treatment. The separation and purification of the product may be performed by using a separation and purification method used for a normal protein, and as described above in the present specification, for example, the product can be separated and purified by appropriately selecting and combining a chromatography such as affinity chromatography, and the like. In addition, in a case where the product is an antibody, the titer of the antibody can also be measured with a commercially available analytical instrument such as Cedex Bio manufactured by F. Hoffmann-La Roche, Ltd.

Examples of the column that is used for affinity chromatography include a protein A column and a protein G column. Examples of the chromatography other than affinity chromatography include ion exchange chromatography, hydrophobic chromatography, gel filtration, reverse phase chromatography, and adsorption chromatography. The chromatography can be carried out using liquid phase chromatography such as high performance liquid chromatography (HPLC) or fast protein liquid chromatography (FPLC).

It is noted that it is also possible to modify the product or partially remove a peptide thereof by allowing an appropriate polypeptide modifying enzyme to act on the useful substance before or after purification. As the polypeptide modifying enzyme, for example, trypsin, chymotrypsin, lysyl endopeptidase, protein kinase, or glucosidase is used.

The product that is produced according to the present invention can be used, for example, for a biopharmaceutical product, or regenerative medicine.

The present invention will be more specifically described using the following examples; however, it is not limited by the examples.

Examples

<Establishment of antibody-producing cell>

A vector including a nucleic acid sequence encoding rituximab, denosumab, blosozumab, anti-hMUC1, or emicizumab was constructed, and each constructed vector was introduced into CHO-DG44 cells to prepare CHO cells A, B, C, D, and E that each expressed an antibody. The construction of the vector and the introduction thereof into the cell were carried out according to Example 2 of JP2016-517691A. As described above, CHO cells producing monoclonal antibodies were prepared and used in the following experiment.

[Perfusion culture experiment]

In Examples and Comparative Examples, experiments were carried out using the cell culture device shown in FIG. 1. CHO cells that produce antibodies in perfusion culture were cultured to a target viable cell density (for example, 120 $\times$ $10^6$ cells/ml), and the culture was continued while maintaining the cell density. The culture was continued for a total of 34 days unless the hollow fiber MF membrane was clogged during the culture period. In a case where the hollow fiber MF membrane was clogged, the culture was stopped.

Specifically, the following experiments were performed.

1.3 L of a culture medium is charged into a culture tank having a total capacity of 3 L (Biostat (registered trademark)) manufactured by Sartorius AG. The culture media A to E shown in Table 1 are culture media adjusted with reference to the exchange culture media in Table 2 of JP2000-517188A, and are culture media to which a poloxamer was added to have the concentration shown in Table 1. Kolliphor (registered trademark) P188 Geismar manufactured by BASF SE was used as the poloxamer in the culture medium. CHO cells were seeded in the above culture medium at $0.5 \times 10^6$ cells/ml. At a stirring rotation speed of 160 rpm, 38 ml/min of air and 2 ml/min of $CO_2$ from the upper surface, and $O_2$ from the bottom surface were automatically controlled and supplied from a sparger installed in the culture tank so that the oxygen concentration in the culture solution was 80%.

After culturing for 2 days after seeding, the cell culture solution was continuously filtered with ATF2 manufactured by Repligen Corporation, and the recovery liquid was recovered while continuously supplying a culture medium at a perfusion ratio of 0.6 vvd.

Further, on the 5th day, the perfusion ratio was changed to the predetermined perfusion ratio shown in Table 1, and the perfusion ratio was maintained thereafter.

Thereafter, in a case where the cell density reached the target viable cell density shown in Table 1, the cell bleeding was carried out while a part of the culture solution was drained so that the cell density was maintained. The cell bleeding was continuously carried out automatically such that the cell density was constant using a FUTURA sensor manufactured by ABER Instruments Ltd. while monitoring the electrostatic capacity of the culture solution.

In this case, a plurality of levels were performed by changing the cells used, the antibody produced, the culture medium, the culture conditions, and the like as shown in Examples and Comparative Examples of Table 1.

Thereafter, the culture was continued, and the evaluation described below was carried out.

In a case of the 50 L culture, the culture was carried out under the same conditions as those in the 1.3 L culture, except that 50 L of a culture medium was charged into a culture tank of Hyperforma 50 manufactured by Thermo Fisher Scientific Inc., the stirring rotation speed was set to 250 rpm, the setting was such that an upper surface air of 0.475 L/min and $CO_2$ of 0.025 L/min, and ATF4 manufactured by Repligen Corporation was used.

<Automatic control method for cell bleeding>

In a case where the cell bleeding was automatically controlled, the cell bleeding was continuously carried out automatically using a FUTURA sensor manufactured by ABER Instruments Ltd. while monitoring the electrostatic capacity of the culture solution. Specifically, the measurement was carried out by the following method.

(1) An electrostatic capacity serving as a target is set.
(2) A weight control value of the culture tank is set.
(3) The electrostatic capacity of the culture solution is measured at a period of 0.1 s or less.
(4) A pump for draining the culture solution is driven at a rate of 0.3 vvd to 3 vvd in a case where the electrostatic capacity of the culture solution exceeds the target.
(5) A culture medium is automatically supplied into the culture tank while the weight of the culture tank is measured so that the liquid amount of the culture solution is roughly kept constant.
(6) The pump for draining the culture solution is stopped in a case where the electrostatic capacity of the culture solution is smaller than the target by 0.01% or more.

<Measurement and evaluation method>

(1) Measurement of cell density

The culture solution in the culture tank was extracted and subjected to measurement using a Cell Viability Analyzer Vi-cell XR manufactured by Beckman Coulter, Inc. It is noted that for the Vi-cell software, Vi-cell XR2.04 was used, and the parameters at the time of measurement were set as follows.

Min diameter: 6 $\mu$m

Max diameter: 50 $\mu$m

Dilution: In a case where the cell density was $100 \times 10^5$ cells/mL or less, the sample was not diluted, and the measurement was carried out at a Dilution of 1. In a case where the cell density was $100 \times 10^5$ cells/mL or more, the sample was diluted 10 times, and the measurement was carried out at a Dilution of 10.

Cell brightness: 85%

Cell sharpness: 100

Viable cell spot brightness: 75%

Viable cell spot area: 5%

Minimum circularity: 0

Decluster degree: Medium

The measurement was carried out once a day by the above method.

(2) Measurement of antibody concentration in culture solution and permeated solution

A sample of the culture solution was extracted from the culture tank, and the cells were filtered with Whatman (pore size: 0.2 $\mu$m, diameter: 25 mm) manufactured by Cytiva. Then, the filtrate was measured with Cedex Bio manufactured by F. Hoffmann-La Roche, Ltd.

The permeated solution was measured with Cedex Bio manufactured by F. Hoffmann-La Roche, Ltd. after sampling.

The measurement was carried out once a day by the above method.

(3) Measurement of poloxamer concentration in culture solution

The culture solution sample was extracted from the culture tank, the cells were filtered with Whatman (pore size: 0.2 $\mu$m, diameter: 25 mm) manufactured by Cytiva, and the filtrate was collected.

1.17 g of maleic acid (guaranteed reagent, manufactured by FUJIFILM Wako Pure Chemical Corporation) is added to 50 ml of deuterium oxide (manufactured by Cambridge Isotope Laboratories, Inc.) and dissolved.

0.4 ml of heavy water in which 20, 50, 100, or 200 mg of Kolliphor P188 (manufactured by BASF SE) has been dissolved is added to 1.6 ml of heavy water containing maleic acid, and the mixture is measured by 1H NMR (400 MHz, Ascend 400, manufactured by Bruker Corporation) with 64 accumulations. A calibration curve is drawn at peak 1 (near 0.8 to 1.1 ppm) and peak 2 (near 3.4 to 3.5 ppm) of the poloxamer with reference to 6.2 ppm, which is the peak of the maleic acid.

0.4 ml of the culture supernatant is added to 1.6 ml of heavy water containing maleic acid, and the mixture is measured by 1H NMR with 64 accumulations to perform quantification from the area ratio of peak 1 or peak 2 to the maleic acid. Either peak 1 or peak 2 is used depending on the level of impurities.

(4) Evaluation of hollow fiber MF membrane clogging

As the evaluation of hollow fiber MF membrane clogging, the measurement of "1. Reciprocating pressure of ATF" was performed as the evaluation of clogging of the flow channel portion of the hollow fiber, and the measurement of "2. MF membrane permeation rate of antibody" was performed as the evaluation of clogging of the pore portion of the hollow fiber.

1. Reciprocating pressure of ATF (evaluation of clogging of flow channel portion of hollow fiber)

A pressure gauge for measuring the pumping and suction pressure of the ATF diaphragm pump was installed below the membrane of the ATF, that is, on the upper portion of the diaphragm ($P_{In}$), a pressure gauge for measuring the outlet pressure of the membrane of the ATF was installed above the membrane of the ATF ($P_{Re}$), and a pressure gauge for measuring the permeation pressure was installed on the permeate side of the ATF ($P_{Pe}$). Each pressure gauge was zero-point calibrated with the atmosphere before driving the ATF, and then the ATF was driven. The maximum value of $P_{In}$ during one reciprocation of the diaphragm was defined as $P_{In-max}$, the minimum value of $P_{In}$ was defined as $P_{In-min}$, the maximum value of $P_{Re}$ was defined as $P_{Re-max}$, and the minimum value of $P_{Re}$ was defined as $P_{Re-min}$, and these values were recorded every day. $P_{In-max} - P_{Re-max}$ was used as the reciprocating pressure of the ATF. In a case where the reciprocating pressure of the ATF exceeded 5 psi, clogging was determined to have occurred and the culture was interrupted.

2. MF membrane permeation rate of antibody (evaluation of clogging of pore portion of hollow fiber)

The MF membrane permeation rate of the antibody was evaluated as $CP \div CB \times 100$ [%] in a case where the antibody concentration of the culture solution was defined as CB and the antibody concentration of the permeated solution was defined as CP.

<Evaluation determination>

Evaluation determination was performed on the last culture day with (1) reciprocating pressure of ATF, (2) MF membrane permeation rate of antibody, and (3) titer, and the overall evaluation was performed according to each result.
In a case where the hollow fiber MF membrane reached the clogging criterion, the culture was interrupted at that point, and that day was defined as the last culture day. In a case where the hollow fiber MF membrane was not clogged, the culture was continued until the 34th day, and that day was defined as the last culture day. Table 2 shows the measurement results on the last culture day.

(1) Reciprocating pressure of ATF

D: In a case where the reciprocating pressure of the ATF reached the clogging criterion until the 34th day
C: In a case where the reciprocating pressure of the ATF is 4 psi or more and 5 psi or less on the 34th day
B: In a case where the reciprocating pressure of the ATF is 3 psi or more and less than 4 psi on the 34th day
A: In a case where the reciprocating pressure of the ATF is less than 3 psi on the 34th day

(2) MF membrane permeation rate of antibody

C: In a case where the MF membrane permeation rate of the antibody on the last culture day is less than 50%
B: In a case where the MF membrane permeation rate of the antibody on the last culture day is 50% or more and 70% or less
A: In a case where the MF membrane permeation rate of the antibody on the last culture day exceeds 70%

(3) Titer

The titer was defined as a value obtained by multiplying the antibody concentration in the permeated solution by the perfusion ratio on that day, and the unit thereof was g/L/day.

C: Titer is less than 1.0 g/L/day
B: Titer is 1.0 g/L/day or more and 1.5 g/L/day or less
A: In a case where the titer exceeds 1.5 g/L/day

(4) Overall evaluation

D: In a case where there is a D determination
C: In a case where there are no D determination and one or more C determinations
B: In a case where all are B determination or more and there is one or less A determination
A: In a case where all are B determination or more and there are two or more A determinations

Table 2 shows measurement results and evaluations of each of Examples and each of Comparative Examples.
FIG. 2 shows the measurement results of the viable cell density for Example 1 and Comparative Example 1.

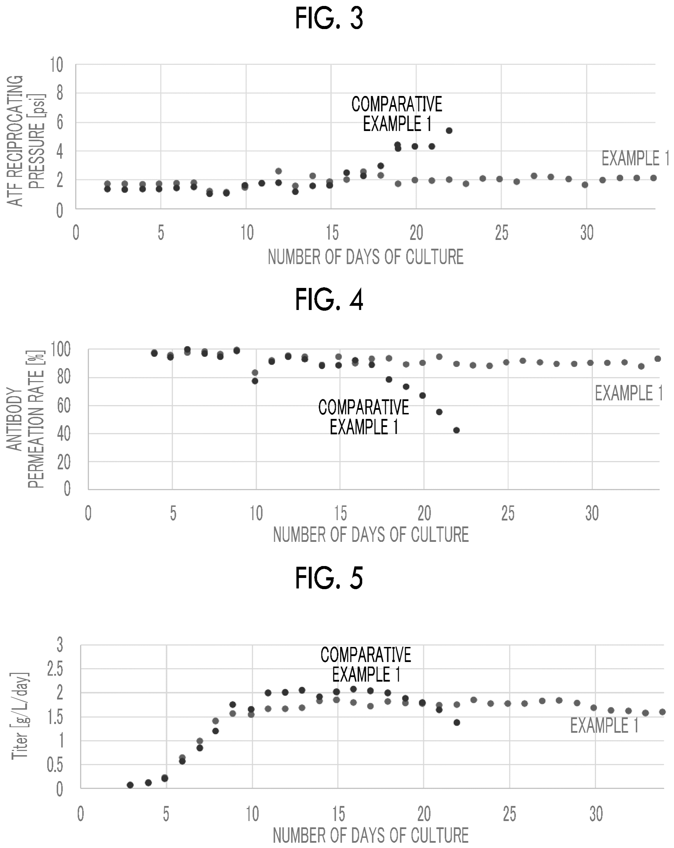

FIG. 3 shows the measurement results of the reciprocating pressure of the ATF for Example 1 and Comparative Example 1.
FIG. 4 shows the measurement results of the antibody permeation rate for Example 1 and Comparative Example 1.
FIG. 5 shows the measurement results of the titer for Example 1 and Comparative Example 1.

[Table 1]

EP 4 722 378 A1

| | Culture conditions | | | | | | | | | | | |
|---|---|---|---|---|---|---|---|---|---|---|---|---|
| | Amount of culture solution | Cell | | | | | | | | Culture medium | | Perfusion ratio after 5th day | Target viable cell density |
| | | Type of CHO cell | Antibody to be produced | | | | | | Culture medium No | Poloxamer concentration | | |
| | [L] | | Antibody No | Name | Subclass | Light chain structure | SAP value S | Ratio V% of valine residue of L chain variable region | | g/L | vvd | M cells/ml |
| Example 1 | 1.3 | A | Antibody 1 | Rituximab | IgG1 | κ | 383 | 5.8 | Culture medium A | 5 | 1.2 | 120 |
| Example 2 | 1.3 | A | Antibody 1 | Rituximab | IgG1 | κ | 383 | 5.8 | Culture medium B | 6 | 1.2 | 120 |
| Example 3 | 1.3 | A | Antibody 1 | Rituximab | IgG1 | κ | 383 | 5.8 | Culture medium C | 10 | 1.2 | 120 |
| Example 4 | 1.3 | A | Antibody 1 | Rituximab | IgG1 | κ | 383 | 5.8 | Culture medium D | 3.5 | 1.2 | 120 |
| Example 5 | 1.3 | A | Antibody 1 | Rituximab | IgG1 | κ | 383 | 5.8 | Culture medium E | 2.5 | 1.2 | 140 |
| Example 6 | 1.3 | A | Antibody 1 | Rituximab | IgG1 | κ | 383 | 5.8 | Culture medium E | 5 | 0.8 | 120 |
| Example 7 | 1.3 | B | Antibody 2 | Denosumab | IgG2 | κ | 374 | 3.8 | Culture medium A | 5 | 1.2 | 120 |
| Example 8 | 1.3 | C | Antibody 3 | Blosozumab | IgG4 | κ | 377 | 5.8 | Culture medium A | 5 | 1.2 | 120 |
| Example 9 | 1.3 | D | Antibody 4 | Anti-hMUC1 | IgG1 | λ | 394 | 8.7 | Culture medium B | 5 | 1.2 | 90 |
| Example 10 | 1.3 | D | Antibody 4 | Anti-hMUC1 | IgG1 | λ | 394 | 8.7 | Culture medium B | 5 | 2 | 120 |
| Example 11 | 50 | A | Antibody 1 | Rituximab | IgG1 | κ | 383 | 5.8 | Culture medium A | 5 | 1.2 | 120 |
| Example 12 | 1.3 | E | Antibody 5 | Emicizumab | IgG4 | κ | 376 | 3.8 | Culture medium A | 5 | 1.2 | 120 |
| Comparative Example 1 | 1.3 | D | Antibody 4 | Anti-hMUC1 | IgG1 | λ | 394 | 8.7 | Culture medium A | 5 | 1.2 | 120 |
| Comparative Example 2 | 1.3 | D | Antibody 4 | Anti-hMUC1 | IgG1 | λ | 394 | 8.7 | Culture medium B | 10 | 1.2 | 120 |
| Comparative Example 3 | 50 | D | Antibody 4 | Anti-hMUC1 | IgG1 | λ | 394 | 8.7 | Culture medium B | 5 | 1.2 | 110 |
| Comparative Example 4 | 1.3 | A | Antibody 1 | Rituximab | IgG1 | κ | 383 | 5.8 | Culture medium B | 10 | 1.2 | 170 |

15

[Table 2]

EP 4 722 378 A1

| | Measurement results | | | | | | | | | | | Evaluations | | | |
| | Culture solution | | | | | | Permeated solution | | | Pressure | | | | | |
| | Viable cell density | Poloxamer concentration P | Antibody concentration C | 1.5P + 3C − 0.93S | Ratio V of valine residue of L chain variable region | P + 3.5C + 1.9V | Antibody concentration | Antibody permeation rate | Titer (antibody concentration in permeated solution × perfusion ratio) | Reciprocating pressure of ATF | Last culture day | Reciprocating pressure of ATF | Antibody permeation rate | Titer | Overall evaluation |
| | M cells/ml | g/L | g/L | | % | | g/L | % | g/L/day | psi | | | | | |
| Example 1 | 118 | 5 | 1.5 | 368 | 5.8 | 21 | 1.4 | 91 | 1.6 | 2.1 | 34 | A | A | A | A |
| Example 2 | 120 | 6 | 1.5 | 370 | 5.8 | 22 | 1.4 | 95 | 1.7 | 2.8 | 34 | A | A | A | A |
| Example 3 | 121 | 10 | 1.7 | 376 | 5.8 | 27 | 1.6 | 94 | 1.9 | 4.2 | 34 | C | A | A | C |
| Example 4 | 118 | 3.5 | 2.3 | 368 | 5.8 | 23 | 1.6 | 71 | 1.9 | 2.3 | 34 | A | A | A | A |
| Example 5 | 138 | 2.5 | 3.6 | 371 | 5.8 | 26 | 1.8 | 50 | 2.2 | 4 | 34 | C | B | A | C |
| Example 6 | 122 | 5 | 2.7 | 372 | 5.8 | 25 | 1.8 | 67 | 1.4 | 4.1 | 34 | C | B | A | C |
| Example 7 | 118 | 5 | 1.3 | 359 | 3.8 | 17 | 1.2 | 92 | 1.4 | 2.5 | 34 | A | A | B | A |
| Example 8 | 118 | 5 | 1.1 | 361 | 5.8 | 20 | 1 | 91 | 1.2 | 2.3 | 34 | A | A | B | A |
| Example 9 | 90 | 5 | 1.2 | 378 | 8.7 | 26 | 0.8 | 63 | 0.9 | 4.7 | 34 | C | B | C | C |
| Example 10 | 120 | 5 | 1.2 | 378 | 8.7 | 26 | 0.9 | 75 | 1.8 | 4.8 | 34 | C | A | A | C |
| Example 11 | 120 | 5 | 1.6 | 368 | 5.8 | 22 | 1.4 | 90 | 1.7 | 2 | 34 | A | A | A | A |
| Example 12 | 121 | 5 | 1 | 360 | 3.8 | 16 | 0.92 | 92 | 1.1 | 2.2 | 34 | A | A | B | A |
| Comparative Example 1 | 117 | 5 | 2.9 | 382 | 8.7 | 32 | 1.2 | 42 | 1.4 | 5.4 | 22 | D | C | B | D |
| Comparative Example 2 | 122 | 10 | 3.6 | 392 | 8.7 | 39 | 1.7 | 47 | 2 | 6.2 | 18 | D | C | A | D |
| Comparative Example 3 | 112 | 5 | 2.3 | 381 | 8.7 | 30 | 1.2 | 52 | 1.4 | 5.3 | 30 | D | B | B | D |
| Comparative Example 4 | 166 | 10 | 2.8 | 380 | 5.8 | 31 | 1.9 | 68 | 2.3 | 5.2 | 33 | D | B | A | D |

Explanation of References

1: sparger air supply pipe
2: air supply pipe
3: culture medium supply pipe
4: gas exhaust pipe
5: gas exhaust filter
6: sampling tube
7: bleeding tube
8: electrostatic capacity sensor
9: dissolved oxygen sensor
10: culture container
11: sparger
12: stirring blade
13: perfusion device
14: flow of permeated solution
15: culture medium supply pump
16: delivery pump
17: pressure gauge
18: diaphragm pump
19: foam

**Claims**

1. A production method for a product, comprising:

    culturing cells in a culture solution,
    wherein, in a case where a poloxamer concentration in the culture solution is denoted by P (g/L),
    a product concentration in the culture solution is denoted by C (g/L),
    a hydrophobicity index SAP value of the product is denoted by S, and
    a ratio of a valine residue of an L chain variable region of the product is denoted by V (%),
    at least one of

$$1.5P + 3C + 0.93S \leq 378 \quad \text{(Expression 1)}$$

    or

$$P + 3.5C + 1.9V \leq 29 \quad \text{(Expression 5)}$$

    is satisfied.

2. The production method for a product according to claim 1,
    wherein the poloxamer concentration P (g/L) satisfies

$$1.5 \leq P \leq 15 \quad \text{(Expression 2)}.$$

3. The production method for a product according to claim 1,
    wherein the product concentration C (g/L) satisfies

$$0.2 \leq C \leq 10 \quad \text{(Expression 3)}.$$

4. The production method for a product according to claim 1,
    wherein S, which is the hydrophobicity index SAP value, satisfies

$$300 \leq S \leq 394 \text{ (Expression 4).}$$

**5.** The production method for a product according to claim 1,
wherein the ratio V (%) satisfies

$$0 \leq V \leq 9 \text{ (Expression 6).}$$

**6.** The production method for a product according to claim 1,
wherein the culturing is perfusion culture.

**7.** The production method for a product according to claim 6,
wherein the perfusion culture includes a cell separation step of separating the cells by a filtration membrane.

**8.** The production method for a product according to claim 7,
wherein the cell separation step is performed by an ATF method which is an alternating tangential flow.

**9.** The production method for a product according to claim 6,
wherein, in the perfusion culture, the product is produced in a certain period while maintaining a target viable cell density substantially constant after reaching the viable cell density in the culturing.

**10.** The production method for a product according to claim 9,
wherein the viable cell density maintained constant is $30 \times 10^6$ cells/ml or more and $400 \times 10^6$ cells/ml or less.

**11.** The production method for a product according to claim 6,
wherein a perfusion ratio in the perfusion culture is 0.3 or more and 3.0 or less.

**12.** The production method for a product according to any one of claims 1 to 11,
wherein a period of the culturing is 14 days or more and 200 days or less.

**13.** The production method for a product according to any one of claims 1 to 11,
wherein the cell is a CHO cell.

**14.** The production method for a product according to any one of claims 1 to 11,
wherein the product is a protein.

**15.** The production method for a product according to claim 14,
wherein the protein is an antibody.

**16.** The production method for a product according to claim 15,
wherein a light chain structure of the antibody is a $\kappa$ type.

# FIG. 1

# FIG. 2

## FIG. 3

## FIG. 4

## FIG. 5

# EP 4 722 378 A1

## INTERNATIONAL SEARCH REPORT

| | International application No. |
|---|---|
| | **PCT/JP2024/019989** |

### A. CLASSIFICATION OF SUBJECT MATTER

*C12P 21/08*(2006.01)i
FI:   C12P21/08

According to International Patent Classification (IPC) or to both national classification and IPC

### B. FIELDS SEARCHED

Minimum documentation searched (classification system followed by classification symbols)

C12P21/08

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| A | JP 2021-505182 A (AMGEN INC.) 18 February 2021 (2021-02-18) | 1-16 |
| A | JP 2007-525984 A (DSM IP ASSETS B.V.) 13 September 2007 (2007-09-13) | 1-16 |
| A | Poloxamer 188 EMPROVE (Registered Trademark) EXPERT 細胞培養グレード, [online]. MERCK. Technical Brief. [retrieved on 05 August 2024]. 2022. Retrieved from the Internet: <URL: https://www.sigmaaldrich.com/deepweb/assets/sigmaaldrich/product/documents/245/636/tb1249jp-mk.pdf>, non-official translation (Poloxamer 188 EMPROVE (Registered Trademark) EXPERT Cell culture grade) | 1-16 |
| A | WO 2023/058723 A1 (CHUGAI SEIYAKU KABUSHIKI KAISHA) 13 April 2023 (2023-04-13) | 1-16 |

☐ Further documents are listed in the continuation of Box C.   ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **05 August 2024** | **13 August 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

International application No.

**PCT/JP2024/019989**

| Patent document cited in search report | | | Publication date (day/month/year) | Patent family member(s) | | | Publication date (day/month/year) |
|---|---|---|---|---|---|---|---|
| JP | 2021-505182 | A | 18 February 2021 | US | 2021/0163592 | A1 | |
| | | | | WO | 2019/118426 | A1 | |
| | | | | EP | 3724229 | A1 | |
| JP | 2007-525984 | A | 13 September 2007 | US | 2008/0131934 | A1 | |
| | | | | WO | 2005/095578 | A1 | |
| | | | | EP | 1720972 | A1 | |
| WO | 2023/058723 | A1 | 13 April 2023 | CA | 3233924 | A1 | |
| | | | | CN | 118019549 | A | |

Form PCT/ISA/210 (patent family annex) (July 2022)

# EP 4 722 378 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021505182 A
- JP 2016517691 A

- JP 2000517188 A

**Non-patent literature cited in the description**

- **NARESH CHENNAMSETTY**. Design of therapeutic proteins with enhanced stability. *Proc Natl Acad Sci USA.*, 21 July 2009, vol. 106 (29), 1937-42

- **SHAUN D. BLACK**. Development of hydrophobicity parameters to analyze proteins which bear post- or cotranslational modifications. *Analytical Biochemistry*, 15 February 1991, vol. 193 (1), 72-82